# EUROPEAN PATENT SPECIFICATION

(11) **EP 3 219 257 B1**
(45) Date of publication and mention of the grant of the patent: **13.02.2019**
(21) Application number: 16161196.7
(22) Date of filing: 18.03.2016
(51) Int. Cl.: A61B 5/22, A61B 5/16, A61B 5/00

(54) **DEVICE FOR MEASURING A PRESSURE FORCE**
VORRICHTUNG ZUR MESSUNG EINER ANPRESSKRAFT
DISPOSITIF POUR MESURER UNE FORCE DE PRESSION

(43) Date of publication of application: 20.09.2017
(73) Proprietor: Bromm, Boris, 63150 Bad Homburg (DE)
(72) Inventor: Bromm, Burkhart, 24105 Kiel (DE)
(74) Representative: Maiwald Patent- und Rechtsanwaltsgesellschaft mbH

(56) References cited:
- EP-A1- 2 359 747
- EP-A1- 2 529 786
- WO-A1-95/14430
- WO-A1-2013/006639
- WO-A2-2009/052100
- US-A1- 2012 232 422
- US-A1- 2013 023 799
- US-A1- 2013 046 205

## Description

### Field of the invention

The invention generally relates to medical devices. In particular, the invention relates to a device for measuring a pressure force, a system for determining a sensation experienced by a person, the use of a device for measuring a pressure force and a method for determining a sensation experienced by a person.

### Background of the invention

There exist different types for measuring and recording sensations and emotions of human beings in general. In particular, it can be distinguished between three categories to measure sensations and emotions of a person. The first possibility is to measure an emotional experience of a person. This kind of measurement refers to questionnaires combined with scales. All kinds of images and Figures are being used such as drawings, smileys and numbers representing the current emotional experience of the person. The person indicating his emotional experience has to rationalize and translate his experience into the given scheme of the scales. Recently, much software and many Apps have been provided as digital versions of such measurement of emotional experiences.

The second possibility is to measure sensations and emotions on a physiological basis. There are numerous physiological appearances that are being used as indicators in the measurement of emotions and sensations such as heart rate, blood pressure, skin conductance, lid closure reflex, peripheral blood flow, respiration, perspiration and even brain waves and brain scans. The third possibility to measure sensations and emotions of a person refers to the expression behaviour of the person. This concerns the analysis of facial mimics of the person. In particular, a landscape of facial mimics of the person can be designed. The individual facial mimic of the person indicating an emotion is being matched with this landscape to objectify the quality and intensity of the emotion.

The measurement and recording of sensations and emotions plays an important role for instance in the area of pain. The measurement and recording of pain is of high importance in the development and testing of new drugs as well as in the medical treatment of pain patients. It is subject to constant and intensive research. Physiological measurement is being used to measure pain objectively. This means that in the laboratory, brain images under experimental pain are used to investigate and quantify main states of pain experienced by the person. Furthermore, in the therapy of pain, scales and questionnaires are used to measure the subjective pain experience of a person, trying to track and objectify the subjective pain experience. For example, a slight control with a scale from 0 to 10 is being operated by the patient, wherein "0" stands for "no pain" and "10" stands for "severe pain". The person which, in this case, is the patient moves the slight control to a number on the scale in order to objectify the pain experience in the dialogue with the physician. For small children, smileys instead of numbers can be used on the scale. Such smileys may comprise sad faces, neutral faces and happy faces. In order to record and track the pain measurement over a longer period of time, it is being noted into a pain diary by the patient.

US 2013/0046205 A1 describes a device for detecting and measuring pain felt by a person. The device comprises a pressure or force sensor, a hollow body having an outer sleeve and an inner space. The outer sleeve of the hollow body is embodied in such a way that it can be at least partially surrounded by a hand. The hollow body is filled with a non-gaseous elastic material.

US 2013/0023799 A1 describes an apparatus for detecting hand force or hand pressure. The apparatus includes a longitudinally extending hollow body having an upper fixed end part and a lower fixed end part. The hollow body further includes a flexible outer cover. A pressure measuring apparatus transmits the pressure from the outer cover via a pressure transmitter to the pressure measuring apparatus.

WO 2009/052100 A2 describes an apparatus for use in studying a patient's bowel. A pain input detector, a pain transducer and a processor are provided. Output data may be generated to reflect perceived patient pain.

WO 95/14430 describes a method for determination of the weight of a human body. Therein, force or pressure exerted by the foot of the human body on supporting means of the foot is measured by sensing means incorporated in a shoe.

### Summary of the invention

It is an object of the present invention to improve the evaluation of an emotion or sensation experienced by a person.

This object is achieved by the subject-matter of the independent claims. Further exemplary embodiments are evident from the dependent claims and the following description.

According to a first aspect of the present invention, a device for measuring a pressure force is provided. The device comprises a hand-held body made of an elastic material. The elastic material may be natural or synthetic rubber. The device further comprises a sensor unit which is completely enclosed by the hand-held body such that a force applied by a person onto the hand-held body is transmitted via the elastic material of the hand-held body to the sensor unit. The sensor unit is configured for measuring a pressure force which is transmitted through the elastic material to the sensor unit and which is caused by the force applied onto the hand-held body by the person. The sensor unit is configured for generating data corresponding to the measured pressure force.

In other words, the device may be adapted for measuring a pressure force applied by the person such that an evaluation of a sensation or emotion experienced by the person is facilitated. The person may, in this case, be a patient for which medical data or data relevant for the physical health of the patient can be obtained by the device. The force which is applied onto the hand-held body by the person represents a current emotion or a sensation of the person. In particular, the extent or level of the force may be an indicator for the sensation or emotion experienced by the person. Such an experienced sensation may for instance be a pain, a transient itching, an irregular anxiety, a general comfort or discomfort, a joy or a sympathy, stress, spasms, birth pangs, hunger or thirst perceived by the person. The device therefore provides the possibility to subjectively evaluate the current sensation or emotion experienced by the person in that the device or, in particular, the pressure unit measures the force applied onto the hand-held body by the person wherein the force is characteristic of a specific experienced sensation or emotion of the person.

The hand-held body may be small enough such that it can be carried in the hand of the person and such that the person can exert the pressure force onto the hand-held body by means of only one hand, for example by squeezing the hand-held body. This means that the person, which is considered to be the patient from which medical data is required, may enclose the hand-held body with his hand and in particular with his fingers and his palm such that the person may compress the hand-held body. The elastic material may transmit the force applied onto the hand-held body by the person such that the transmitted force is exerted on the sensor unit in the form of a pressure force. The sensor unit is then able to detect this transmitted pressure force which represents the force applied onto the hand-held body by the person. This pressure force can then be stored on a storage medium, e.g. a chip.

In a preferred embodiment, the hand-held body comprises a longitudinal shape. The hand-held body may have the form of an egg, a cylinder, a bowl, a banana or the like. In general, every form or shape of the hand-held body is possible as long as the person is able to carry or hold this hand-held body by means of only one hand and such that the person is able to squeeze or compress the hand-held body by means of only one hand. This is advantageous since the device can be carried along with the person using the device. Furthermore, using an elastic material does not only provide a reliable transmission of the pressure force, but also provides a pleasant and comfortable feeling for the person using the device.

The sensor unit is further adapted to generate data corresponding to the measured pressure force such that this data may be used to identify or evaluate the emotion or sensation experienced by the person.

According to an embodiment of the invention, an interface configured to transmit the generated data corresponding to the measured pressure force to an external evaluation system is provided.

In this manner, it is possible to transmit the generated data to another system on which this data may be analyzed. The interface may be integrated into the hand-held body such that, for example, a connection to the external evaluation system can be provided. The interface may for instance be a USB interface. However, other types of interfaces for data transfer are possible. The interface may be additionally used to recharge a battery located inside the hand-held body.

According to another embodiment of the invention, the interface is configured to wirelessly transmit the data corresponding to the measured pressure force.

A wireless transmission may be achieved by Bluetooth or a wireless local area network for example. A wireless transmission is advantageous because the generated data corresponding to the measured pressure force indicating a current sensation or emotion experienced by the patient can be transferred via a long distance, for example to the physician which can analyze this generated data in order to evaluate and establish a clinical picture of the patient.

According to another embodiment, which is not a part of the invention, the hand-held body comprises an opening in which the interface is located such that the interface is connectable to the external evaluation system using a wire-bound connection.

In other words, the hand-held body may be directly connected to a personal computer or to a workstation on which the generated data corresponding to the measured pressure force can be analyzed. In this manner, the data may be reliably read out from a storage medium, e.g. a chip, integrated into the hand-held body on which the generated data can be stored.

Alternatively, the hand-held body does not have an opening. In other words, the interface is completely surrounded by the elastic material of the hand-held body. In this case the interface is a wireless interface which is advantageous because it allows the hand-held body to be perfectly closed, and therefore the device is waterproof and washable. In particular, a battery located inside the hand-held body can be recharged wirelessly. In this case the hand-held body does not need an opening and is waterproof and washable since the battery can be completely enclosed by the elastic material of the hand-held body. Therefore, both data transfer as well as recharging the battery may be conducted wirelessly. However, the device may also be designed such that a wirebound recharge of the battery or a wirebound data transfer is possible, for example with an opening in the elastic material of the hand-held body.

According to another embodiment of the invention, a storage medium which is configured to store the data corresponding to the measured pressure force is provided. The storage medium is at least partially enclosed by the hand-held body.

Using a storage medium is advantageous because it makes the measurement independent from access to internet and other electronic devices such as computers. Hence, the measurement can take place anytime and anywhere and the person measuring his or her emotion is not disturbed by any kind of evaluation, recording or visualization of the measurement. In other words, a measurement may be conducted even if the device is not connected to the internet or to an external system. This is of particular importance, for example in situations where the user of the device is located in a region where the access to the internet or an external system is not possible or where this access is disturbed. In this case, the measurement can be stored on the storage medium without directly transmitting the generated data corresponding to the measured pressure force to the external evaluation system. The stored data can be transmitted to the external evaluation system for further analysis at a later time, for example when the user of the device has access to the internet or to the external evaluation system.

Preferably, the storage medium is fully enclosed into the hand-held body such that the person which is using the hand-held body only touches the elastic material of the device. The storage medium may be a chip on which the measured data can be stored and from which the stored data can be read out. The storage medium may store such data over a pre-determinable period of time. It is possible that reading out data from the storage medium is password-protected such that only authorized persons have access to the data corresponding to the measured pressure force and therefore to data which indicate a current sensation of a patient. This is advantageous because medical data representing a medical condition of a patient is very sensitive data to which only authorized personnel, e.g. a physician, should have access.

According to the invention, the device further comprises an output unit for providing an acoustic and/or haptic signal to the person at a pre-determinable time. The output unit is at least partially enclosed by the hand-held body.

The acoustic signal may for instance be a bleep or a similar sound which can be noticed by the person. The haptic signal may for instance be a vibration of the hand-held body. Such an acoustic or haptic signal may remind the person to use and in particular to squeeze the hand-held body in order to provide data of a current sensation or emotion experienced by the person. These signals may be triggered at pre-determinable times in order to periodically collect data from the person. The fact that the hand-held body may be carried by the person is advantageous because the device can always be used by the person in different situations during daytime or night time. For example, if the device is not in use, it can be carried in the pocket since it is small enough in that it may be compressed by a person's hand.

According to the invention, the device comprises a pin-shaped member which is completely enclosed by the hand-held body. The sensor unit comprises sensors which are attached to an outside of the pin-shaped member.

The pin-shaped member may be adapted to the form of the hand-held body such that the pin-shaped member has a smaller diameter than the hand-held body and the pin-shaped member is located inside the hand-held body. In other words, the pin-shaped member may be surrounded by the elastic material of the hand-held body. The pin-shaped member may comprise a metallic or synthetic material. Preferably, the pin-shaped member comprises a plastic material which is advantageous since it reduces the weight of the device and it does not disturb wireless data transfer.

The skilled person may choose from different elastic materials. For example, unsaturated rubbers like natural polyisoprene rubber, synthetic polyisoprene rubber, polybutadiene rubber, chloroprene rubber, butyl rubber, halogenated butyl rubbers, styrene-butadiene rubber, nitrile rubber, hydrogenated nitrile rubbers or saturated rubbers like ethylene propylene rubber, epichlorohydrin rubber, polyacrylic rubber, silicone rubber, fluorosilicone rubber, fluoroelastomers, perfluoroelastomers, polyether block amides, chlorosulfonated polyethylene, ethylene-vinyl acetate or other types of elastomers like thermoplastic elastomers, resilin, elastin, polysulfide rubber and elastolefin can be chosen. But also other materials may be used as long as the chosen material transmits the applied force of the user towards the sensor unit of the device.

In general, it should be noted that "elastic material" represents a material which can be deformed by a hand of the person, e.g. by muscular strength.

Furthermore, the sensor unit may comprise a plurality of sensors which is attached to the pin-shaped member on an outer surface of the pin-shaped member. The sensors may have the shape of a thin foil which is adapted to a contour of the pin-shaped member. Preferably, the sensors are glued or bonded to the outer surface of the pin-shaped member.

It is possible that only one sensor is attached to the pin-shaped-member, for example by wrapping a rectangular sensor foil around the pin-shaped member. The sensor foil may be bonded to the outer surface of the pin-shaped member by an adhesive.

The rectangular sensor foil may also have an elongated cut such that a plurality of those elongated sensor foils are equidistantly attached in juxtaposition on the outer surface of the pin-shaped member. This is advantageous because it reduces the quantity of sensor foil needed and therefore also reduces the costs.

According to another embodiment of the invention, the sensors comprise a curved surface which is adapted to a contour of the pin-shaped member.

The sensors may have the shape of a thin foil. It may be wrapped around the pin-shaped member. In particular, several sensors may be located on the curved surface of the pin-shaped member in juxtaposition. This means that the sensors may be wrapped around the pin-shaped member side by side. This aspect will be described in more detail in the description of the drawing.

According to the invention, the sensors are attached to a lateral surface of the pin-shaped member.

The pin-shaped member may have the form of a cylinder wherein the lateral surface, e.g. the curved surface of the cylinder, comprises the sensors. The sensors may be attached to the lateral surface and/or wrapped around the lateral surface in juxtaposition. There may be one, two, three, four or more sensors which are located on the lateral surface of the pin-shaped member. In this manner, the pressure force which is transmitted via the elastic material of the hand-held body may be reliably detected.

According to another embodiment of the invention, the sensor unit is configured for measuring an intensity of the measured pressure force. The intensity of the measured pressure force corresponds to a level or extent of the sensation perceived by the person.

This means that the higher a pressure force is, the higher a level of a sensation perceived by the person may be. The pressure force measured by the sensor unit is thus an indicator for the sensation perceived by the person. The sensation may be a pain, a transient itching, an irregular anxiety, a general comfort or discomfort, a joy or a sympathy, stress, spasms, birth pangs, hunger or thirst perceived by the person. For example, the sensation may be a discomfort accompanied by heart attacks.

Therefore, the elastic material of the hand-held body must be suitable to transmit small pressure forces as well as high pressure forces such that many different levels of sensations perceived by the person can be detected. For example, the elastic material of the hand-held body is an elastomer such as rubber, for example. This rubber may be a synthetic or a natural rubber.

According to another aspect of the present invention, a system for determining a sensation experienced by a person is provided. The system comprises the device as described above as well as an external evaluation system configured to receive the data corresponding to the measured pressure force via the interface. The external evaluation system is configured for determining a sensation experienced by the person based on the received data.

For example, the external evaluation system is configured for determining a sensation perceived by the person wherein the sensation is selected from a group, the group comprising a pain, a transient itching, an irregular anxiety, a depression, a comfort or a discomfort, a joy or a sympathy, stress, spasms, birth pangs, hunger or thirst. The system may further be configured for tracking or monitoring the appearance of certain thoughts of the person. In particular, the external evaluation system is configured for determining a period of time and an intensity of a thought appeared within the mind of this person based on the measured pressure force. In other words, the thought of the person can be determined based on the evaluated sensation of the person. Likewise, the system may also be configured for tracking or monitoring certain habits of the person.

According to an embodiment of the invention, the external evaluation system, which is a part of the system for determining a sensation experienced by a person, is configured for monitoring an effectiveness of a pharmaceutical administered to the person.

Hence, it is possible to evaluate whether a certain pharmaceutical, e.g. a pain medicine, is working effectively based on the sensation, e.g. pain, experienced by a test person. Moreover, it is possible to evaluate whether a certain pharmaceutical has a positive or a negative influence on the person, e.g. the patient or the test person, wherein this evaluation is based on the determined sensation experienced by the person and therefore also based on the data corresponding to the measured pressure force.

As a result it is not only possible to determine the sensation experienced by a person in general but also to analyze how specific therapeutic activities or administered pharmaceuticals affect the person. Since it is possible to collect data with the help of the device over a long period of time, the influence of a pharmaceutical on the person can be reliably analyzed.

According to another aspect of the invention, the use of a device as described above for evaluating a current sensation of a person is provided. Furthermore, the device can be used for evaluating the effectiveness of pharmaceuticals, e.g. a pain medicine, administered to the person.

According to another aspect of the present invention, a method for determining a sensation experienced by a person is provided. In a step of the method, a force is applied onto a hand-held body by a person squeezing the hand-held body. In another step, a pressure force onto the hand-held body is measured by means of a sensor unit wherein the pressure force is transmitted via the elastic material to the sensor unit and wherein the pressure force is caused by the force applied by the person. The sensor unit is completely enclosed by the hand-held body. In another step, data corresponding to the measured pressure force is generated by means of the sensor unit. This method of the present invention may also be seen as a method of measuring a pressure force.

According to an embodiment of the invention, a further step comprises the transmission of data corresponding to the measured pressure force via an interface to an external system by means of a wireless. In a further step, the sensation experienced by the person is evaluated wherein the evaluation is based on the data corresponding to the measured pressure force. This evaluation is conducted by means of the external system.

According to another embodiment of the invention, a recording of the evaluated sensation experienced by the person is conducted in a further step. In yet another step, a visualization of the evaluated sensation experienced by the person is provided, for example at a work station.

Further aspects and advantages of the present invention are described in the following:
By means of the device, it is possible to subjectively evaluate a sensation of a person. In general, the device provides an introspection of the person who presses the hand-held body. However, many sources of errors of methods using questionnaires and scales or scale controls can be bypassed by the inventive device. For example, the emotion does not have to be translated into numbers or smileys on a scale. If the scales become more dimensional, e.g. strength and length of the pain, they become more complex and confusing. The person, e.g. the patient, has to spend a lot of concentration on filing out the questionnaire or interpreting the scales. Furthermore, there is a time gap between the emotional experience and filing out the questionnaire or operating the control scale. In contrast, the inventive device can be carried along in a pocket and the measuring of emotions and sensations can be fully integrated in a discrete manner in the day-to-day environment. This will result in a more fragment or a much more compliant measurement delivering more precise data. By means of the device the measurement of the emotion can take place the very moment of its occurrence, anywhere and anytime.

The inventive device or the inventive system bypasses the intermediate of a questionnaire, a scale or a scale control. The emotion and the measurement can be kept inside the person such that the person is enabled to fully concentrate at any time and everywhere on his pain by pressing or squeezing the device, e.g. applying a force on the hand-held body of the device. Neither eyes nor ears nor speech nor the ability to write or tip on smileys is necessary for the measurement and the recording of the emotion or sensation. The measurement takes place inwardly and therefore emphasizes the subjective determination of a sensation or emotion of the person.

Physiological methods of measuring emotions have altogether the disadvantage that they aim to find an objective way to measure emotions and sensations. However, emotions and sensations are subjective experiences such that in practice it is the subjective experience that matters and not the objective physiological appearance. A pain patient, for instance, has to be treated considering his subjective pain experience, not considering the result of objective measurements. Besides, the physiological methods of measuring emotions require complex and costly systems and machinery. Those methods are not designed to measure emotions constantly and over a long period of time in a day-to-day environment. In contrast, the inventive device allows a measurement which is constant over a long period of time and which can be used in almost every situation in a day-to-day environment. Therefore, the inventive device including the hand-held body is a very practicable means for reliably determining the subjective sensation or emotion of the person. The inventive device and the inventive system provide the possibility that the time and the strength of the squeezing or pressing of the hand-held body can be measured and recorded. Afterwards, the generated data can be analyzed in order to conclude a current sensation or emotion of the person. The elastic material of the hand-held body can be imagined as a rubber egg for example since it is preferably egg-shaped. Inside the hand-held body, the pin-shaped member may be located in the form of a hard plastic tube. Force sensors are attached on the outside of the plastic tube. In the inside of the plastic tube, a chip, a battery, which may be recharged wireless or wirebound, and an interface for a wireless or a wire-bound connection may be located. If a person squeezes the hand-held body, e.g. the rubber egg, force sensors measure the time, the length and the strength of the squeeze and the chip records the measured data. The hand-held body may be shaped in the form of a ball, a cylinder, a pineapple, a banana or the like. Preferably, the hand-held body may have a longitudinal shape such that the shape of the pin-shaped member follows the shape of the hand-held body. Data transmission from the hand-held body to the external system may be conducted wirelessly, e.g. via Bluetooth, or via cable. In particular, the measured data can be read out from the chip by means of a computer as external system. The external system, e.g. the computer, may show the course of the measurement via a graphical user interface like a monitor, for example.

The measurement of emotions and sensations is no longer disturbed by rational processes such as filing questionnaires, diaries, scales and scale controls. Moving scales or touching little smileys on a mobile phone is no longer necessary. Such procedures require paper, a pen, a clock and a lot of discipline. They are altogether an unreasonable demand for a patient suffering pain. These drawbacks can be overcome by the inventive device and the inventive system. In particular, the strength of the sensation or the level of sensation is directly translated into mechanical pressure. In fact, the eyes are no longer necessary at all in the process of measurement and recording of emotions. The process of measurement of emotions is now completely inward. Therefore, the person that measures its emotions, for example the pain patient, can fully concentrate on his or her emotional experience and not on the translation or interpretation of it. However, the pain strength or the level of pain is directly translated into pressure. Furthermore, the inventive device or the device as part of the inventive system provides the possibility to measure, record and monitor at any time anywhere in a comfortable and discrete manner sensations and emotions perceived by the person. No paper and no internet are necessary. It is very likely that this will increase the compliance of the patient to measure and record his emotions or sensations in a sustainable manner. Furthermore, more precise data will be collected.

The inventive device further has a therapy effect. For example, clenching a fist or squeezing something or pressing in general is the natural reaction of a person experiencing strong emotions. For example, if a patient suffers pain at a dentist or if a person experiences joy at a soccer game. Squeezing a piece of soft rubber helps to deal with the emotion in an expressive and intensive way. Furthermore, it can be done discrete.

The inventive device allows an uncomplicated measurement and recording of emotions not only in terms of strong and weak, but also in terms of quality. For instance, a short stingy pain can be measured and recorded with a short strong pressing of the hand-held body. The slowly growing pain that fades away after some moments can be measured and recorded with a slowly increasing and decreasing pressing of the hand-held body.

Finally, the device as part of the inventive system for determining a sensation experienced by the person fosters patient enablement. In other words, it involves the person experiencing the sensation, e.g. a patient suffering chronic pain, actively into the therapy and promotes dialogue between the patient and his medical doctor, e.g. a pain specialist. When measuring takes place at a medical centre, the patient and the physician can look one another in the eye and communicate while the patient squeezes the elastomeric hand-held body according to his acute pain experience. No intermediate such as moving a scale control or clicking on the smileys of an App stands in between and disturbs the conversation. This also applies for investigations with an experimentally induced pain.

### Brief description of the drawings

- Fig. 1: schematically shows a device for measuring a pressure force according to an embodiment of the invention.
- Fig. 2: shows a device for measuring a pressure force according to another embodiment of the invention.
- Fig. 3: shows the device for measuring a pressure force according to another embodiment of the invention.
- Fig. 4A: shows a system for determining a sensation experienced by a person according to an embodiment of the invention.
- Fig. 4B: shows a system for determining a sensation experienced by a person according to another embodiment of the invention.
- Fig. 5: shows a flow diagram a method for determining a sensation experienced by a person according to an embodiment of the invention.

### Detailed description of the drawings

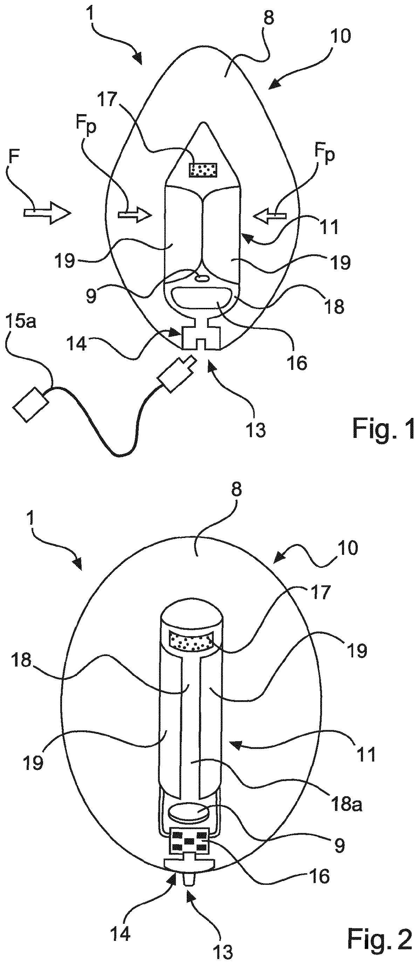

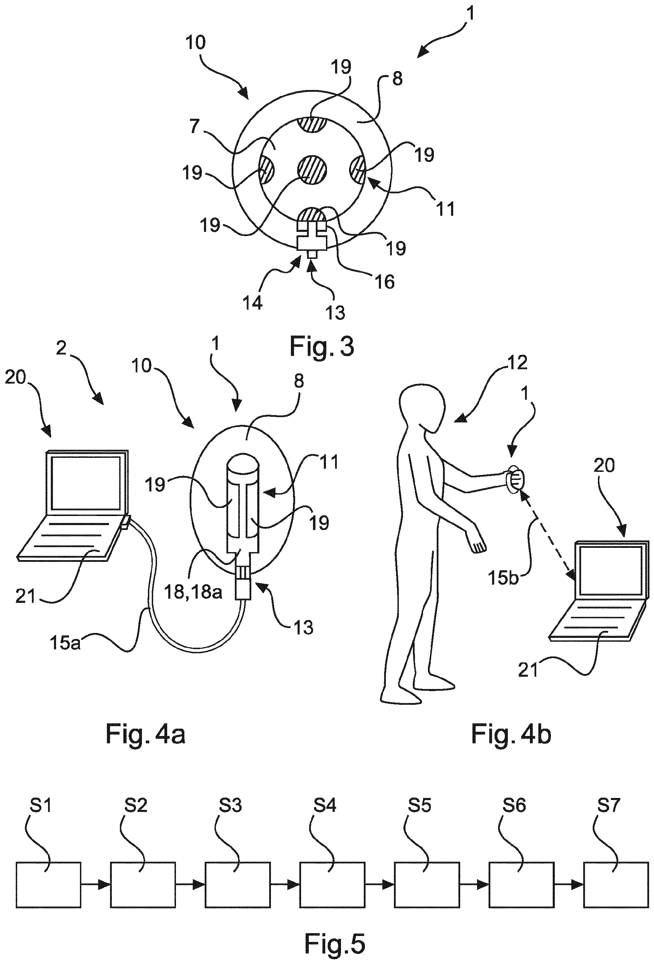

Fig. 1 shows a device 1 for measuring a pressure force. The device 1 comprises a hand-held body 10 made of an elastic material 8 such as rubber. The device 1 further comprises a sensor unit 11 which is completely enclosed by the hand-held body 10. In other words, the elastic material 8 which may be deformed by a person squeezing the hand-held body 10 is surrounding or enclosing the sensor unit 11. However, the sensor unit 11 is completely enclosed by the hand-held body 10 such that a force F applied by a person, which is not shown in Fig. 1, onto the hand-held body 10 is transmitted via the elastic material of the hand-held body 10 to the sensor unit 11. The sensor unit 11 is configured for measuring a pressure force Fₚ which is transmitted to the sensor unit 11 and which is caused by the force F applied onto the hand-held body 10 by the person. The sensor unit 11 is configured for generating data corresponding to the measured pressure force Fₚ. The force F is the result of a squeezing initiated by the person that holds the hand-held body 10 by means of only one hand. The pressure force Fₚ is transmitted via the elastic material 8 of the hand-held body 10 to the sensor unit 11 which itself comprises sensors 19. Preferably, the plurality of sensors 19 is located inside the hand-held body 10. The hand-held body 10 further comprises an interface 13 in an opening 14 for connecting the device 1 to an external system which is also not shown in Fig. 1. This connection may be provided by a wirebound connection 15a or a wireless connection as will be described in Fig. 4B. The hand-held body 10 of the device 1 comprises a pin-shaped member 18 which has the sensors 19 attached to a lateral surface of the pin-shaped member 18. The pin-shaped member 18 has an elongated form as shown in Fig. 1 which is adapted to an elongated form of the hand-held body 10. For example, the hand-held body 10 has the shape of an egg. Other designs of the hand-held body 10 are possible. In particular, every form which allows a person to squeeze or compress the hand-held body 10 by means of only one hand represents a suitable design for the hand-held body 10. The sensors 19 may have the form of a foil which is at least partly wrapped around the lateral surface of the pin-shaped member 18. The hand-held body 10 generally comprises two main parts. The first main part is defined by the elastic material 8 and the second main part is defined by the pin-shaped member 18 which itself comprises the interface 13, the sensor unit 11 as well as other elements like for example an output unit 17, a battery 9 and a storage medium 16. On the storage medium 16, data representing the pressure force Fₚ which is measured by the sensor unit 11 can be stored. The battery 9 delivers energy, in particular electrical energy for the operation of the sensor unit 11, the storage medium 16 and, in case an output unit 17 is present like shown in Fig. 1, also for the output unit 17. The battery 9 may be charged in a wireless or wirebound manner. The output unit 17 may be adapted to provide an acoustic or a haptic signal. The elastic material 8 of the hand-held body 10 may at one side of the hand-held body 10 have an opening 14 at which the interface 13 is located. In this manner, it is possible to connect the device 1 to an external system by means of a wire-bound connection 15a.

It should be noted that Fig. 1 shows a cross-sectional view through the elastic material 8 as well as a perspective view of the inner a part of the hand-held body 10 which inner a part comprises the pin-shaped member 18, the sensor unit 11, the output unit 17, the battery 9, the storage medium 16 as well as the interface 13.

Fig. 2 also shows a cross-sectional view through the elastic material 8 of the hand-held body 10 of the device 1. The inner part is again visualized by means of a perspective view. In particular, the inner part comprises the pin-shaped member 18 in the form of a cylinder which has a lateral surface 18a. Sensors 19 of the sensor unit 11 are attached to the lateral surface 18a of the pin-shaped member 18. In Fig. 2, it can be recognized that the sensors 19 have the form of a foil which for each sensor is at least partially wrapped around the lateral surface 18a of the pin-shaped member 18. The pin-shaped member 18 may also comprise an output unit 17 which may be integrated into the pin-shaped member 18 as well as a battery 9 which may also be integrated into the pin-shaped member 18. The storage medium 16 may for instance be a chip which is also integrated into the pin-shaped member 18 or which is attached to the pin-shaped member 18 such that the chip is located between the pin-shaped member 18 and the interface 13 as shown in Fig. 2. Furthermore, the opening 14 provides the possibility to connect the interface 13 to an external system not shown in Fig. 2. In other words, the opening 14 provides a recess for receiving the interface 13 in the elastic material 8.

Fig. 3 shows another embodiment of the device for measuring a pressure force by means of a cross-sectional view through the elastic material 8 as well as a perspective view of the inner part comprising a spherically shaped member 7 to which the sensor unit 11 is attached.

According to the invention, several sensors 19 of the sensor unit 11 are attached to an outer surface of the spherically shaped member 7.

Several forms or shapes of the hand-held body 10 are possible. However, it is required that the hand-held body 10 can be held and squeezed by only one hand of a person.

The device 1 further comprises a storage medium 16 on which the data corresponding to the measured pressure force Fₚ can be stored. The storage medium may be at least partly enclosed by the spherical member 7.

Fig. 4A, which is not a part of the claimed invention, shows a system 2 for determining a sensation experienced by a person. The system 2 comprises a device 1 which comprises the hand-held body 10 as described in Figs. 1 to 3. Fig. 4A shows the device 1 with a pin-shaped member 18 and the lateral surface 18a on which the sensors 19 are attached. The sensors 19 are part of the sensor unit 1. The pin-shaped member 18 with the sensor unit 11 is surrounded by the elastic material 8 wherein the pin-shaped member 18 with its integrated parts and the surrounding elastic material 8 form the hand-held body 10. An external evaluation system 20, which for instance is a personal computer 21, is connected to the device 1. This connection is based on a cable bound connection 15a as shown in Fig. 4. The device 1 comprises an interface 13 to which the external evaluation system 20 can be connected by means of the wire-bound connection 15a.

Fig. 4B, however, shows that the connection between the device 1 and the external evaluation system 20 can also be based on a wireless connection 15b. This is advantageous especially where the sensation of the person 12 has to be analyzed over a long distance. In other words, it is possible that the physician can monitor a current sensation of the person 12 by means of the external evaluation system 20 which for instance is a personal computer 21. The person 12 can carry the device 1, for example, in a pocket when the device 1 is not in use. If a measurement of a current sensation of the person 12 is required, an acoustic or haptic signal may be supplied to the person 12 via the output unit 17 at a predeterminable time. The person 12 can then squeeze the device 1, e.g. the hand-held body, such that a pressure force indicating the current sensation of the person 12 can be evaluated by the physician even over a long distance between the physician and the person 12.

Fig. 5 shows a flow diagram for a method for determining a sensation experienced by the person 12. In a step S1 of the method, a force F is applied onto a hand-held body 10, e.g. of an elastic material 8, by a person 12 squeezing the hand-held body 10. In another step S2, a sensor unit 11 measures a pressure force Fₚ onto the hand-held body 10 which is transmitted to the sensor unit 11 via the elastic material 8 and which is caused by the force F applied by the person 12. The sensor unit 11 is completely enclosed by the hand-held body 10. In another step S3, data corresponding to the measured pressure force Fₚ is generated by means of the sensor unit 11. In a further step S4 of the method, data corresponding to the measured pressure force Fₚ is transmitted via an interface 13 to an external system 20 by means of a wireless 15a or wire-bound 15b connection. In another step S5, a sensation experienced by the person 12 is evaluated based on the data corresponding to the measured pressure force Fₚ by means of the external system 20. In another step S6, the evaluated sensation experienced by the person 12 is recorded. In yet another step S7, a visualization of the evaluated sensation experienced by the person 12 is conducted, for example via a graphical user interface.

While the invention has been illustrated and described in detail in the drawings and the foregoing description, such illustration and description are to be considered illustrative and exemplary and non-restrictive; the invention is not limited to the disclosed embodiments. Other variations to the disclosed embodiments can be understood and effected by those skilled in the art and practising the claimed invention, from a study of the drawings, the disclosure, and the appended claims. In the claims, the word "comprising" does not exclude other elements, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope of protection.

## Claims

1. Device (1) for measuring a pressure force, comprising:
a hand-held body (10) made of an elastic material (8);
a sensor unit (11);
a pin shaped member (18) comprising a hard metallic or synthetic material;
**characterized in that**
the sensor unit (11) is completely enclosed by the hand-held body (10) such that a force (F) applied by a person (12) onto the hand-held body (10) is transmitted via the elastic material (8) of the hand-held body (10) to the sensor unit (11);
wherein the sensor unit (11) is configured for measuring a pressure force (Fₚ) which is transmitted to the sensor unit (11) and which is caused by the force (F) applied onto the hand-held body (10) by the person (12); and
wherein the sensor unit (11) is configured for generating data corresponding to the measured pressure force (Fₚ);
wherein the pin-shaped member (18) is completely enclosed by the hand-held body (10);
wherein the sensor unit (11) comprises sensors (19) which are attached to a lateral surface (18a) of the pin-shaped member (18).

2. Device (1) according to claim 1, comprising:
an interface (13) configured to transmit the generated data corresponding to the measured pressure force (Fₚ) to an external evaluation system (20).

3. Device (1) according to claim 2,
wherein the interface (13) is configured to wirelessly transmit the data corresponding to the measured pressure force (Fₚ).

4. Device (1) according to any one of the preceding claims, comprising:
an output unit (17) for providing an acoustic and/or haptic signal to the person (12) at a predeterminable time;
wherein the output unit (17) is completely enclosed by the hand-held body (10).

5. Device (1) according to claim 1,
wherein the sensors (19) comprise a curved surface which is adapted to a contour of the pin-shaped member (18).

6. Device (1) according to any one of the preceding claims,
wherein the sensor unit (11) is configured for measuring an intensity of the measured pressure force (Fₚ), wherein the intensity of the measured pressure force (Fₚ) corresponds to a level of a sensation perceived by the person (12).

7. Device (1) for measuring a pressure force, comprising:
a hand-held body (10) made of an elastic material (8);
a sensor unit (11);
**characterized in that** the device (1) further comprises
an inner part comprising a spherically shaped member (7);
wherein the sensor unit (11) is completely enclosed by the hand held body (10) such that a force (F) applied by a person (12) onto the hand-held body (10) is transmitted via the elastic material (8) of the hand-held body (10) to the sensor unit (11);
wherein the sensor unit (11) is configured for measuring a pressure force (Fₚ) which is transmitted to the sensor unit (11) and which is caused by the force (F) applied onto the hand-held body (10) by the person (12); and
wherein the sensor unit (11) is configured for generating data corresponding to the measured pressure force (Fₚ);
wherein the sensor unit (11) comprises sensors (19) being attached to an outer surface of the spherically shaped member (7).

8. Device (1) according to any one of the preceding claims, comprising:
a storage medium (16) configured to store the data corresponding to the measured pressure force (Fₚ);
wherein the storage medium (16) is at least partly enclosed by the hand-held body (10).

9. System (2) for determining a sensation experienced by a person (12), comprising:
a device (1) according to any one of the preceding claims;
an external evaluation system (20) configured to receive the data corresponding to the measured pressure force (Fₚ) via an interface (13);
wherein the external evaluation system (20) is configured for determining a sensation experienced by the person (12) based on the received data.

10. System (2) according to claim 9,
wherein the external evaluation system is configured for monitoring an effectiveness of a pharmaceutical administered to the person.

11. Method for determining a sensation experienced by a person (12), comprising the steps:
Applying a force (f) onto a hand-held body (10) by a person (12) squeezing the hand-held body (10, S1);
Measuring by means of a sensor unit (11) a pressure force (Fₚ) onto the hand-held body (10) which is transmitted to the sensor unit (11) and which is caused by the force (F) applied by the person (12), wherein the sensor unit (11) is completely enclosed by the hand-held body (10, S2); and
Generating data corresponding to the measured pressure force (Fₚ) by means of the sensor unit (11, S3);
wherein the pin-shaped member (18) comprises a hard metallic or synthetic material;
wherein a pin-shaped member (18) is completely enclosed by the hand-held body (10);
wherein the sensor unit (11) comprises sensors (19) which are attached to a lateral surface (18a) of the pin-shaped member (18).

12. Method for determining a sensation experienced by a person (12), comprising the steps:
Applying a force (f) onto a hand-held body (10) by a person (12) squeezing the hand-held body (10, S1);
Measuring by means of a sensor unit (11) a pressure force (Fₚ) onto the hand-held body (10) which is transmitted to the sensor unit (11) and which is caused by the force (F) applied by the person (12), wherein the sensor unit (11) is completely enclosed by the hand-held body (10, S2); and
Generating data corresponding to the measured pressure force (Fₚ) by means of the sensor unit (11, S3);
wherein the hand-held body comprises an inner part comprising a spherically shaped member (7);
wherein the sensor unit (11) comprises sensors (19) being attached to an outer surface of the spherically shaped member (7).

13. Method according to claim 11 or 12, comprising the steps:
Transmitting the data corresponding to the measured pressure force (Fₚ) via an interface (13) to an external evaluation system (20) by means of a wireless (15b, S4) connection;
Evaluating a sensation experienced by the person (12) based on the data corresponding to the measured pressure force (Fₚ) by means of the external evaluation system (20, S5).

## Patentansprüche

1. Vorrichtung (1) zur Messung einer Anpresskraft, umfassend:
einen Handhaltekörper (10) aus einem elastischen Material (8);
eine Sensoreinheit (11);
ein stiftförmiges Element (18), das ein hartes metallisches oder synthetisches Material umfasst;
**dadurch gekennzeichnet, dass**
die Sensoreinheit (11) vollständig von dem Handhaltekörper (10) umschlossen ist, sodass eine von einer Person (12) auf den Handhaltekörper (10) ausgeübte Kraft (F) über das elastische Material (8) des Handhaltekörpers (10) auf die Sensoreinheit (11) übertragen wird;
wobei die Sensoreinheit (11) zur Messung einer Anpresskraft (F_{P}) eingerichtet ist, die auf die Sensoreinheit (11) übertragen wird und die durch die von der Person (12) auf den Handhaltekörper (10) ausgeübte Kraft (F) verursacht ist; und
wobei die Sensoreinheit (11) zur Generierung von Daten eingerichtet ist, die der gemessenen Anpresskraft (F_{P}) entsprechen;
wobei das stiftförmige Element (18) vollständig von dem Handhaltekörper (10) umschlossen ist;
wobei die Sensoreinheit (11) Sensoren (19) umfasst, die an einer seitlichen Oberfläche (18a) des stiftförmigen Elements (18) befestigt sind.

2. Vorrichtung (1) nach Anspruch 1, umfassend:
eine Schnittstelle (13) zur Übertragung der generieten Daten, die der gemessenen Anpresskraft (F_{P}) entsprechen, an ein externes Auswertungssystem (20).

3. Vorrichtung (1) nach Anspruch 2,
wobei die Schnittstelle (13) zur drahtlosen Übertragung der Daten eingerichtet ist, die der gemessenen Anpresskraft (F_{P}) entsprechen.

4. Vorrichtung (1) nach einem der vorhergehenden Ansprüche, umfassend:
eine Ausgabeeinheit (17) zur Bereitstellung eines akustischen und/oder haptischen Signals an die Person (12) zu einer vorgebbaren Zeit;
wobei die Ausgabeeinheit (17) vollständig von dem Handhaltekörper (10) umschlossen ist.

5. Vorrichtung (1) nach Anspruch 1,
wobei die Sensoren (19) eine gekrümmte Oberfläche aufweisen, die an eine Kontur des stiftförmigen Elements (18) angepasst ist.

6. Vorrichtung (1) nach einem der vorhergehenden Ansprüche.
wobei die Sensoreinheit (11) zur Messung einer Intensität der gemessenen Anpresskraft (F_{P}) eingerichtet ist, wobei die Intensität der gemessenen Anpresskraft (F_{P}) einem von einer Person (12) wahrgenommenen Empfindungsniveau entspricht.

7. Vorrichtung (1) zur Messung einer Anpresskraft, umfassend:
einen Handhaltekörper (10) aus einem elastischen Material (8);
eine Sensoreinheit (11);
**dadurch gekennzeichnet, dass** die Vorrichtung (1) ferner einen inneren Abschnitt umfasst, welcher ein kugelförmiges Element (7) umfasst;
wobei die Sensoreinheit (11) vollständig von dem Handhaltekörper (10) umschlossen ist, sodass eine von einer Person (12) auf den Handhaltekörper (10) ausgeübte Kraft (F) über das elastische Material (8) des Handhaltekörpers (10) auf die Sensoreinheit (11) übertragen ist;
wobei die Sensoreinheit (11) zur Messung einer Anpresskraft (F_{P}) eingerichtet ist, die auf die Sensoreinheit (11) übertragen wird und die durch die von der Person (12) auf den Handhaltekörper (10) ausgeübte Kraft (F) verursacht ist; und
wobei die Sensoreinheit (11) zur Generierung von Daten eingerichtet ist, die der gemessenen Anpresskraft (F_{P}) entsprechen;
wobei die Sensoreinheit (11) Sensoren (19) umfasst, die an einer Außenfläche des kugelförmigen Elements (7) befestigt sind.

8. Vorrichtung (1) nach einem der vorhergehenden Ansprüche, umfassend:
ein Speichermedium (16), das zur Speicherung der der gemessenen Anpresskraft (F_{P}) entsprechenden Daten eingerichtet ist;
wobei das Speichermedium (16) zumindest teilweise von dem Handhaltekörper (10) umschlossen ist.

9. System (2) zur Bestimmung einer von einer Person (12) erfassten Empfindung, umfassend:
eine Vorrichtung (1) nach einem der vorhergehenden Ansprüche;
ein externes Auswertungssystem (20), das zum Empfangen der der gemessenen Anpresskraft (F_{P}) entsprechenden Daten über eine Schnittstelle (13) eingerichtet ist;
wobei das externe Auswertungssystem (20) zur Bestimmung einer von der Person (12) erfassten Empfindung basierend auf den empfangenen Daten eingerichtet ist.

10. System (2) nach Anspruch 9.
wobei das externe Auswertungssystem (20) zur Überwachung einer Wirksamkeit eines der Person verabreichten Arzneimittels eingerichtet ist.

11. Verfahren zur Bestimmung einer von einer Person (12) erfassten Empfindung, umfassend die Schritte:
Aufbringen (S1) einer Kraft (F) auf einen Handhaltekörper (10) durch eine Person (12), die den Handhaltekörper (10) drückt;
Messen (S2) einer Anpresskraft (F_{P}) auf dem Handhaltekörper (10) mittels einer Sensoreinheit (11), die auf die Sensoreinheit (11) übertragen wird, und die durch die von der Person (12) ausgeübte Kraft (F) verursacht ist, wobei die Sensoreinheit (11) vollständig von dem Handhaltekörper (10) umschlossen ist; und
Generieren (S3) von Daten entsprechend der gemessenen Anpresskraft (F_{P}) mittels der Sensoreinheit (11);
wobei das stiftförmige Element (18) ein hartes metallisches oder synthetisches Material umfasst;
wobei ein stiftförmiges Element (18) vollständig von dem Handhaltekörper (10) umschlossen ist;
wobei die Sensoreinheit (11) Sensoren (19) umfasst, die an einer seitlichen Oberfläche (18a) des stiftförmigen Elements (18) befestigt sind.

12. Verfahren zur Bestimmung einer von einer Person (12) erfassten Empfindung, umfassend die Schritte:
Aufbringen (S1) einer Kraft (F) auf einen Handhaltekörper (10) durch eine Person (12), die den Handhaltekörper (10) drückt;
Messen (S2) einer Anpresskraft (F_{P}) auf dem Handhaltekörper (10) mittels einer Sensoreinheit (11), die auf die Sensoreinheit (11) übertragen wird, und die durch die von der Person (12) ausgeübte Kraft (F) verursacht wird, wobei die Sensoreinheit (11) vollständig von dem Handhaltekörper (10) umschlossen ist; und
Generieren (S3) von Daten entsprechend der gemessenen Anpresskraft (F_{P}) mittels der Sensoreinheit (11);
wobei der Handhaltekörper (10) einen inneren Abschnitt umfasst, der ein kugelförmiges Element (7) umfasst;
wobei die Sensoreinheit (11) Sensoren (19) umfasst, die an einer Außenfläche des kugelförmigen Elements (7) befestigt sind.

13. Verfahren nach Anspruch 11 oder 12, umfassend die Schritte:
Übertragen (S4) der der gemessenen Anpresskraft (F_{P}) entsprechenden Daten über eine Schnittstelle (13) an ein externes Auswertungssystem (20) mittels einer drahtlosen (15b) Verbindung;
Auswerten (S5) einer von der Person (12) erfassten Empfindung mittels des externen Auswertungssystems (20) basierend auf den Daten, die der gemessenen Anpresskraft (F_{P}) entsprechen.

## Revendications

1. Dispositif (1) pour mesurer une force de pression, comprenant :
un corps (10) à saisie manuelle fait en un matériau élastique (8) ;
une unité (11) formant capteur ;
un élément (18) en forme de tige comprenant un matériau métallique ou synthétique dur ;
**caractérisé en ce que**
l'unité (11) formant capteur est complètement entourée par le corps (10) à saisie manuelle de telle sorte qu'une force (F) appliquée par une personne (12) sur le corps (10) à saisie manuelle est transmise à l'unité (11) formant capteur via le matériau élastique (8) du corps (10) à saisie manuelle ;
l'unité (11) formant capteur étant configurée pour mesurer une force de pression (Fₚ) qui est transmise à l'unité (11) formant capteur et qui est provoquée par la force (F) appliquée sur le corps (10) à saisie manuelle par la personne (12) ; et
l'unité (11) formant capteur étant configurée pour générer des données correspondant à la force de pression mesurée (Fₚ) ;
l'élément (18) en forme de tige étant complètement entouré par le corps (10) à saisie manuelle ;
l'unité (11) formant capteur comprenant des capteurs (19) qui sont fixés à une surface latérale (18a) de l'élément (18) en forme de tige.

2. Dispositif (1) selon la revendication 1, comprenant :
une interface (13) configurée pour transmettre les données générées correspondant à la force de pression mesurée (Fₚ) à un système (20) d'évaluation externe.

3. Dispositif (1) selon la revendication 2,
dans lequel l'interface (13) est configurée pour transmettre sans fil les données correspondant à la force de pression mesurée (Fₚ).

4. Dispositif (1) selon l'une quelconque des revendications précédentes, comprenant :
une unité de sortie (17) pour fournir un signal acoustique et/ou un signal haptique à la personne (12) à un moment prédéterminé ;
l'unité de sortie (17) étant complètement entourée par le corps (10) à saisie manuelle.

5. Dispositif (1) selon la revendication 1,
dans lequel les capteurs (19) présentent une surface courbe qui est adaptée à un contour de l'élément (18) en forme de tige.

6. Dispositif (1) selon l'une quelconque des revendications précédentes,
dans lequel l'unité (11) formant capteur est configurée pour mesurer une intensité de la force de pression mesurée (Fₚ), l'intensité de la force de pression mesurée (Fₚ) correspondant à un niveau de sensation perçu par la personne (12).

7. Dispositif (1) pour mesurer une force de pression, comprenant :
un corps (10) à saisie manuelle fait en un matériau élastique (8) ;
une unité (11) formant capteur ;
**caractérisé en ce que** le dispositif (1) comprend en outre
une partie intérieure comprenant un élément (7) de forme sphérique ;
l'unité (11) formant capteur étant complètement entourée par le corps (10) à saisie manuelle de telle sorte qu'une force (F) appliquée par une personne (12) sur le corps (10) à saisie manuelle est transmise par le matériau élastique (8) du corps (10) à saisie manuelle à l'unité (11) formant capteur ;
l'unité (11) formant capteur étant configurée pour mesurer une force de pression (Fₚ) qui est transmise à l'unité (11) formant capteur et qui est provoquée par la force (F) appliquée sur le corps à saisie manuelle (10) par la personne (12) ; et
l'unité (11) formant capteur étant configurée pour générer des données correspondant à la force de pression mesurée (Fₚ) ;
l'unité (11) formant capteur comprenant des capteurs (19) fixés à une surface extérieure de l'élément (7) de forme sphérique.

8. Dispositif (1) selon l'une quelconque des revendications précédentes, comprenant :
un support de stockage (16) configuré pour stocker les données correspondant à la force de pression mesurée (Fₚ) ;
le support de stockage (16) étant au moins partiellement entouré par le corps (10) à saisie manuelle.

9. Système (2) pour déterminer une sensation ressentie par une personne (12), comprenant :
un dispositif (1) selon l'une quelconque des revendications précédentes ;
un système (20) d'évaluation externe configuré pour recevoir les données correspondant à la force de pression mesurée (Fₚ) via une interface (13) ;
le système (20) d'évaluation externe étant configuré pour déterminer une sensation ressentie par la personne (12) en fonction des données reçues.

10. Système (2) selon la revendication 9,
dans lequel le système d'évaluation externe est configuré pour surveiller une efficacité d'un médicament administré à la personne.

11. Procédé pour déterminer une sensation ressentie par une personne (12), comprenant les étapes suivantes :
Appliquer une force (f) sur un corps (10) à saisie manuelle par une personne (12) pressant le corps à saisie manuelle (10, S1) ;
Mesurer au moyen d'une unité (11) formant capteur une force de pression (Fₚ) sur le corps portatif (10) qui est transmise à l'unité (11) formant capteur et qui est provoquée par la force (F) appliquée par la personne (12), l'unité (11) formant capteur étant entièrement entourée par le corps à saisie manuelle (10, S2) ; et
Générer des données correspondant à la force de pression mesurée (Fₚ) au moyen de l'unité formant capteur (11, S3) ;
l'élément (18) en forme de tige comprenant un matériau métallique ou synthétique dur ;
un élément (18) en forme de tige étant complètement entouré par le corps (10) à saisie manuelle ;
l'unité (11) formant capteur comprenant des capteurs (19) qui sont fixés à une surface latérale (18a) de l'élément (18) en forme de tige.

12. Procédé pour déterminer une sensation ressentie par une personne (12), comprenant les étapes suivantes :
Appliquer une force (f) sur un corps (10) à saisie manuelle par une personne (12) pressant le corps à saisie manuelle (10, S1) ;
Mesurer au moyen d'une unité (11) formant capteur une force de pression (Fₚ) sur le corps portatif (10) qui est transmise à l'unité (11) formant capteur et qui est provoquée par la force (F) appliquée par la personne (12), l'unité (11) formant capteur étant entièrement entourée par le corps à saisie manuelle (10, S2) ; et
Générer des données correspondant à la force de pression mesurée (Fₚ) au moyen de l'unité formant capteur (11, S3) ;
le corps à saisie manuelle comprenant une partie intérieure comprenant un élément (7) de forme sphérique ;
l'unité (11) formant capteur comprenant des capteurs (19) fixés à une surface extérieure de l'élément (7) de forme sphérique.

13. Procédé selon la revendication 11 ou la revendication 12, comprenant les étapes suivantes :
Transmettre des données correspondant à la force de pression mesurée (Fₚ) via une interface (13) à un système (20) d'évaluation externe au moyen d'une liaison sans fil (15b, S4) ;
Évaluer une sensation ressentie par la personne (12) en fonction des données correspondant à la force de pression mesurée (Fₚ) au moyen du système d'évaluation externe (20, S5).
